# EUROPEAN PATENT SPECIFICATION

(11) **EP 2 656 071 B1**
(45) Date of publication and mention of the grant of the patent: **12.08.2015**
(21) Application number: 12717473.8
(22) Date of filing: 05.03.2012
(51) Int. Cl.: G01N 33/50

(54) **METHOD TO IDENTIFY LIVER TOXICITY USING METABOLITE PROFILES**
VERFAHREN ZUR IDENTIFIKATION DER LEBERTOXIZITÄT UNTER VERWENDUNG VON METABOLITPROFILEN
PROCÉDÉ POUR IDENTIFIER LA TOXICITÉ DU FOIE AU MOYEN DE PROFILS DE MÉTABOLITES

(30) Priority: 04.03.2011 IN CH06592011
(43) Date of publication of application: 30.10.2013
(73) Proprietor: Strand Life Sciences Private Limited, Karnataka 560024 (IN)
(72) Inventor: SUBRAMANIAN, Kalyanasundaram, Karnataka 560024 (IN); DAS, Sonali, Karnataka 560024 (IN); KUMAR, Rajeev, Karnataka 560024 (IN); RAGHAVAN, Sowmya, Karnataka 560024 (IN); SHAIK, Osman, Shahi, Karnataka 560024 (IN); CHAKRABARTI, Partha, Pratim, Karnataka 560024 (IN); KRISHNAKUMAR, Narasimha, Mandyam, Karnataka 560024 (IN); RAJESWARA, Nalini, Karnataka 560024 (IN); BHAT, Anupama, Rajan, Karnataka 560024 (IN)
(74) Representative: Jansen, Cornelis Marinus
(86) International application number: PCT/IB2012/051025
(87) International publication number: WO 2012/120437

(56) References cited:
- WO-A1-2009/002565
- WO-A1-2010/091290
- US-A1- 2007 043 515
- P. A. DIMAGGIO ET AL: "A Novel Framework for Predicting In Vivo Toxicities from In Vitro Data Using Optimal Methods for Dense and Sparse Matrix Reordering and Logistic Regression", TOXICOLOGICAL SCIENCES, vol. 118, no. 1, 11 August 2010 (2010-08-11), pages 251-265, XP55032075, ISSN: 1096-6080, DOI: 10.1093/toxsci/kfq233
- CHRISTIAN IGEL ET AL: "Covariance matrix adaptation for multi-objective optimization", EVOLUTIONARY COMPUTATION, vol. 15, no. 1, 1 January 2007 (2007-01-01), pages 1-28, XP55032079, DOI: 10.1162/evco.2007.15.1.1
- SUBRAMANIAN KALYANASUNDARAM; RAGHAVAN SOWMYA; RAJAN BHAT ANUPAMA; DAS SONALI; BAJPAI DIKSHIT JYOTI; KUMAR RAJEEV; NARASIMHA MANDYA: "A systems biology based integrative framework to enhance the predictivity of in vitro methods for drug-induced liver injury", EXPERT OPINION ON DRUG SAFETY, vol. 7, no. 6, 1 November 2008 (2008-11-01), pages 647-662, XP9160877, GB ISSN: 1474-0338

## Description

### TECHNICAL FIELD

The present disclosure relates to a methodology that identifies the underlying mechanisms that lead to hepatotoxicity. This is done by using the alterations in cellular metabolite profiles obtained experimentally before and after therapy/drug treatment in combination with a model of liver metabolism. Subsequently, by using a covariance matrix adaptation followed by evolutionary selection, it compares the drug-induced metabolite profiles obtained experimentally with those generated using the *in silico* model, automatically shortlists potential parameters whose alterations could have produced the drug-treated metabolite profile. Values of these parameters are estimated by formulating an optimal control problem to minimize the differences between the model-generated metabolite values and experimentally observed data. The estimated parameters are given as an input to the homeostatic liver model and simulations are carried out, thereby the results providing a mechanistic explanation for the development of toxicity. The activity of the altered parameters identified by the analysis is validated with enzymatic assays or data from published literature to demonstrate the success of this approach.

### BACKGROUND OF THE DISCLOSURE

In the field of the present disclosure, toxic potential of candidate compounds are estimated by monitoring changes in gene expression, protein amounts and/or metabolite profiles. However, the decision about the toxic potential of the compound is ultimately made based on similarity or clustering. Thus a toxicant with hitherto unknown/novel mechanism could potentially be missed if it does not exhibit significant levels of similarity, as measured by standard statistical techniques such as k-means clustering, PCA, etc., with a previously known hepatotoxicant.

DiMaggio et al. [A Novel Framework for Predicting In Vivo Toxicities from In Vitro Data Using Optimal Methods for Dense and Sparse Matrix Reordering and Logistic Regression, Toxicological Sciences, vol. 118, no. 1, 2010-08-11, pages 251-265] describe a biclustering method based on iterative optimal reordering to identify biclusters corresponding to subsets of chemicals to identify which subset of *in vitro* features could be utilized for *in vivo* toxicity prediction. For the calculation, mixed-integer linear optimization (MILP) and logistic regression is combined. US 2007/043515 refers to a method for predicting hepatotoxicity based on differential gene expression, which is calculated by linear discriminant analysis, logistic regression, conjoint analysis, canonical correlation, hierarchical cluster analysis etc. (see US 2007/043515, p. 18,[0189]). WO2010/091290 describes the determination of hepatotoxicity based on measuring and comparing the expression of different biomarkers, wherein random forest analysis was used to calculate the results (see WO 2010/091290, p. 39, I. 13-15). Further, WO 2009/002565 discloses investigation of heptocytoxicity, wherein six or more cellular features are measured, and the response profiles are compared with other response profiles, e.g., in a database. The profiles were determined using a non-parametric

Kolmogorov-Smirnov goodness of fit analysis.

### STATEMENT OF THE DISCLOSURE

Accordingly, the present disclosure relates to a method for predicting liver toxicity of a drug, said method comprising steps of: a) obtaining metabolite profiles from untreated cells and drug-treated cells cells using techniques selected from a group consisting of biochemical assays, mass spectroscopy, NMR or any combinations thereof; b) comparing the metabolite profiles obtained from the untreated cells and the drug-treated cells to determine altered metabolites 1; c) performing Covariance Matrix Adaptive Evolutionary Strategy (CMA-ES) analysis and estimating parameter set responsible for the altered metabolites 1; and d) providing the parameter set as an input to a homeostatic liver model and predicting the toxicity of the drug.

### BRIEF DESCRIPTION OF ACCOMPANYING FIGURES

In order that the disclosure may be readily understood and put into practical effect, reference will now be made to exemplary embodiments as illustrated with reference to the accompanying figures. The figure together with a detailed description below, are incorporated in and form part of the specification, and serve to further illustrate the embodiments and explain various principles and advantages, in accordance with the present disclosure where:

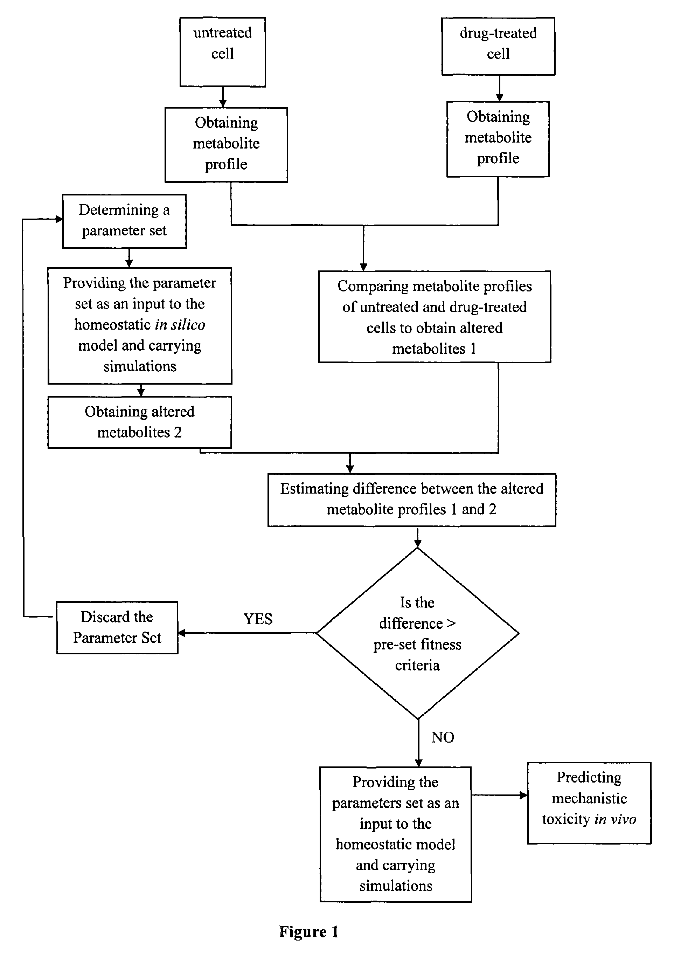

**Figure 1** shows a flow chart of various steps of the disclosure.

### DETAILED DESCRIPTION OF THE DISCLOSURE

The present disclosure relates to a method for predicting liver toxicity of a drug, said method comprising steps of: a) obtaining metabolite profiles from untreated cells and drug-treated cells techniques selected from a group consisting of biochemical assays, mass spectroscopy, NMR or any combinations thereof; b) comparing the metabolite profiles obtained from untreated cells and drug-treated cells to determine altered metabolites 1; c) performing Covariance Matrix Adaptive Evolutionary Strategy (CMA-ES) analysis and estimating parameter set responsible for the altered metabolites 1; and d) providing the parameter set as an input to a homeostatic liver model and predicting toxicity.

In an embodiment of the present disclosure, the CMA-ES analysis comprises steps of: a) determining a parameter set and providing initial values to the parameters in the set; b) providing the parameter set and simulating the homeostatic liver model to obtain altered metabolites 2; c) determining difference between the altered metabolites 1 and the altered metabolites 2; d) selecting the parameter set to be responsible for the altered metabolites 1 if the determined difference is found to be less than a predetermined fitness criterion, or creating a modified parameter set with modified values and repeating above steps b and c if the determined difference is found to be greater than the predetermined fitness criterion.

In another embodiment of the present disclosure, the metabolite profiles are obtained by techniques selected from group comprising biochemical assays, mass spectroscopy, NMR or any combinations thereof.

In yet another embodiment of the present disclosure, the altered metabolites 1 and 2 are obtained by method selected from group consisting of *in-vitro* and *in-silico* methods or a combination thereof.

The present method employs Covariance Matrix Adaptive Evolutionary Strategy (CMA-ES) parameter estimation technique to arrive at toxicity mechanisms *in vivo,* given the experimental metabolite profiles and enables significant reduction in animal usage in toxicological evaluation of new chemical entities.

Understanding the biochemical mechanisms by which drugs interact with the liver is an essential component in preventing drug-induced liver injury (DILI). The present disclosure makes use of the *"in silico* liver metabolism model" as described in Indian Patent Application number 3091/CHE/2008. The said model consists of over 70 metabolites from cytosol, mitochondria, and plasma with over 100 reaction fluxes and over 450 kinetic parameters. Some of the key components of the model are enzymes and transporters of carbohydrate, energy, glutathione, fat, bile salt and bilirubin metabolism. In brief, the *in-silico* liver model is developed by listing of drug- induced organ injuries. This is followed by obtaining molecular mechanisms of toxicity followed by tabulating underlying biochemical pathways of said drugs precipitates organ injury and finally identifying biomolecules, infering biochemical pathways and modeling kinetics of enzymes involved in these pathways to obtain a homeostatic *in silico* model. The model is validated with extensive literature data and with a set of enzyme assays done with known hepatotoxicant. The present disclosure uses this model to perform all computations and simulations.

The method followed by the present disclosure to predict liver toxicity is depicted in figure 1 and is summarized as follows:
First, liver specific cells or cell-lines are treated with drugs or toxicants and metabolite profiles in the cellular extracts are obtained. In the next step, the metabolite profiles of treated and untreated cells (control) are used to find metabolites that are altered (referred as altered metabolites 1). Using a set of parameter values and the homeostatic *in silico* liver model, metabolite profile (referred as altered metabolites 2) that would result upon drug exposure is calculated by simulation. An objective function is then defined based on the differences between the experimentally observed metabolite profile (altered metabolites 1) and the model-generated profile (altered metabolites 2). The function is minimized using a parameter estimation strategy called Covariance Matrix Adaptive Evolutionary Strategy (CMA-ES). This allows us to generate a set of altered parameters that correspond to the changed metabolite profiles obtained upon treatment and represent enzymes, processes or transporters that are affected by the drug or toxicant. The changed parameters are now used as inputs into the homeostatic *in silico* liver model and simulations are run to predict liver toxicity *in vivo.* The said approach is elaborated in greater detail below:

### Steps 1 & 2: Collection of experimental data followed by generation of altered metabolite profiles

Liver specific cells or cell-lines are treated with known doses of drugs or toxicants. Upon treatment, the cells are harvested and cellular extracts prepared. In these extracts, the drug-induced alterations in metabolite profiles can be obtained by using biochemical assays, mass spectrometry, NMR or any other technique for obtaining metabolite profile known in the art. These are then compared with values obtained in the absence of the drug. Metabolites with significant alterations from their untreated values are identified. This generates what is referred to as altered metabolites 1.

### Step 3: Parameter estimation

Metabolites are altered in the liver cells because some enzymes, transporters or processes are altered and operate at different rates compared with the homeostatic rate. To identify the processes that are altered, parameter estimation technique is employed along with the homeostatic *in silico* liver model where enzymes, transporters and processes are modeled as parameters. Covariance matrix adapted evolution Strategy (CMA-ES), is a stochastic, derivative-free numerical optimization method used to solve objective (fitness) functions. It uses real parameter values as solutions with mutation and selection as search operators. New parameter values are determined by using the CMA-ES which overcomes the numerical difficulties associated with derivative based optimization with biological events and to obtain a global minimum. In the present invention, an optimization problem is formulated by defining an objective function to be minimized. This is the error between the *in silico* model predicted altered metabolite profile (referred as altered metabolites 2) and the altered metabolite profile obtained experimentally (altered metabolites 1)

To start the CMA-ES process, an initial population of µ parameter sets are chosen to generate lambda mutants. In an embodiment, µ can be any positive integer. Every parameter set is fed to the *in silico* model to generate metabolite profiles (referred to as altered metabolites 2) by simulation. This altered metabolites 2 is compared with the "altered metabolites 1 and the difference is estimated. If the difference is more than a predefined fitness criterion then the parameter set is discarded. Based on the fitness rankings, the best fitness parameter set becomes the parent for next generation. The said new parameter sets is generated using ES and the CMA is used to compute the values of parameters in the newly generated set. Mutation and cross-over is performed to refine this generation of parameter sets. The modified parameter set is then fed into the *in silico* model to generate new metabolite profiles. The above CMA-ES process is continued till a desired termination criterion is met. The termination criterion is when the difference is less than the predefined fitness criterion. The parameter set obtained by the above explained method is now given as an input to the homeostatic model as described in step 4 below.

In an exemplary embodiment of the present disclosure, an initial population of 40 randomly generated parameter sets with fitness criteria of 10⁻⁸ is used. A normalized weighting factor of 1/C² is used in the fitness function.

### Step 4: Prediction of toxicity mechanisms in vivo

The parameters estimated as described above represent the alterations in enzymes, transporters and processes. To understand how this affects the liver *in vivo,* these parameters are then fed into the liver model and simulations are carried out. The results of this simulation reveal the state of the liver *in vivo,* the user thus effectively obtains predictions of the effects of new drugs *in vivo,* given metabolite profiles of experimental drug treatment.

The above are exemplary modes of carrying out the disclosure and are not intended to be limiting. It will be apparent to those of ordinary skill in the art that modifications thereto can be made without departure from the spirit and scope of the disclosure.

The present disclosure is further elaborated with the help of following examples and associated figures. However, these examples should not be construed to limit the scope of the present disclosure.

### EXAMPLES

To test the applicability of the disclosure and to validate the approach a set of drugs are chosen. These are acetaminophen, diclofenac, cyclosporine, valproic acid and amiodarone. The mechanisms by which these drugs cause toxicity are well documented in the literature.

All metabolite profiles upon drug treatment are obtained experimentally from primary rat hepatocyte cultures. The drug-induced metabolite profiles are then compared with those from untreated cells to obtain altered metabolites 1. The CMA-ES procedure described above is applied wherein, an initial population of 40 randomly generated parameter sets with fitness criteria of 10⁻⁸ is used and the set of likely altered parameters are estimated for the following drugs. Further, a set of biochemical assays are carried out wherever possible to experimentally measure the level of parameter alteration. Tabulated values of the estimated % change in parameters and validation by experimentally observed data (or data from published literature) match well, demonstrating that the present disclosure is capable of providing insights about toxicity as and when it occurs. These results are summarized below.

### EXAMPLE-1

### Studies with Acetaminophen

Acetaminophen is a widely used analgesic whose overdose is often implicated in the development of hepatotoxicity. A total of 19470 parameter set evalutations are required to reach the termination criterion described previously.

The results of the estimated and observed % changes in parameters are shown in table 1.

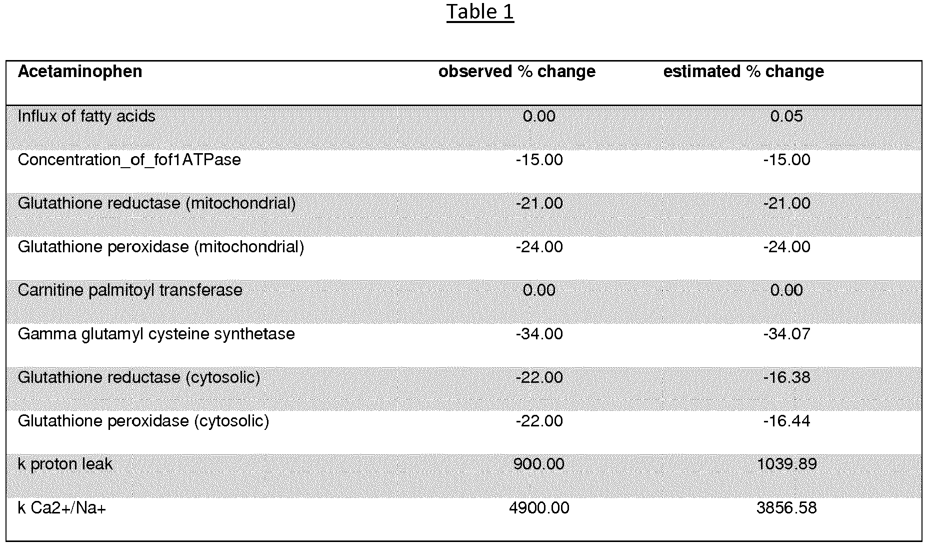

### EXAMPLE-2

### Studies with Amiodarone

Amiodarone is a drug used in clinical managemement of arrhythmia. It is known to cause the development of fatty liver or steatosis which is a well known form of liver injury.

In this case, 26070 parameter set evaluations are needed to reach termination criteria. The results of the estimated and observed % changes in parameters are shown in table 2.

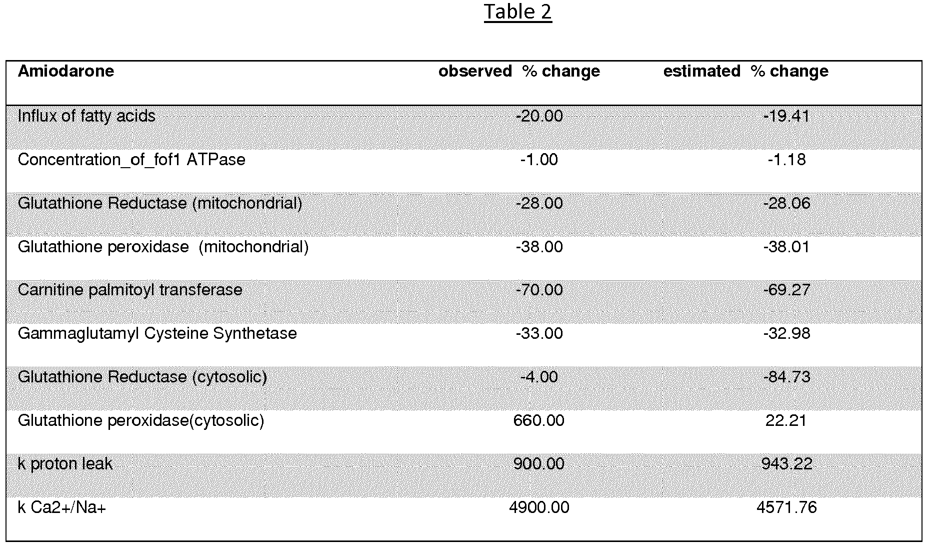

### EXAMPLE-3

### Studies with Cyclosporine

Cyclosporine is an immunosuppressant drug. It is often used under settings of transplantation. It is known to cause cholestatic damage to the liver. In the case of cyclosporine, 27880 parameter set evaluations are needed to reach termination criteria. The results of the estimated and observed % changes in parameters are shown in table 3.

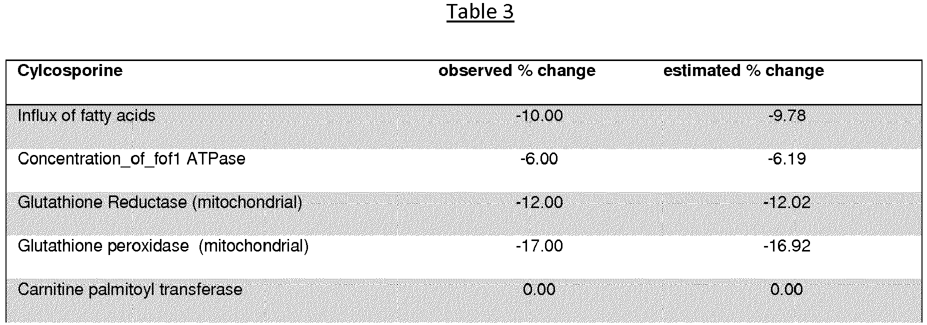

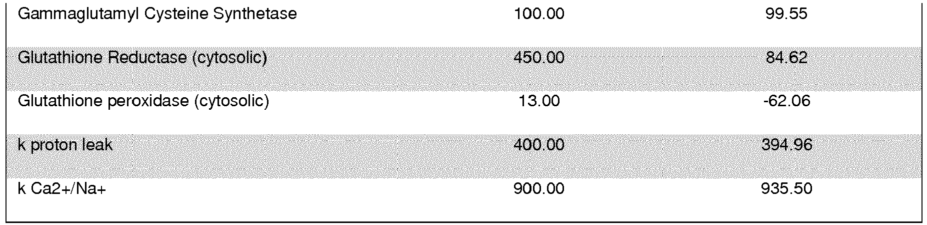

### EXAMPLE-4

### Studies with Diclofenac

Diclofenac is a commonly used NSAID (non-steroidal anti-inflammatory drug) that causes hepatotoxicity by oxidative stress. The observed parameter set is in accordance with literature observations of its mechanism of hepatotoxicity. In this case, 22860 parameter set evaluations are carried out before the termination criterion is satisfied. The results of the estimated and observed % changes in parameters are shown in table 4.

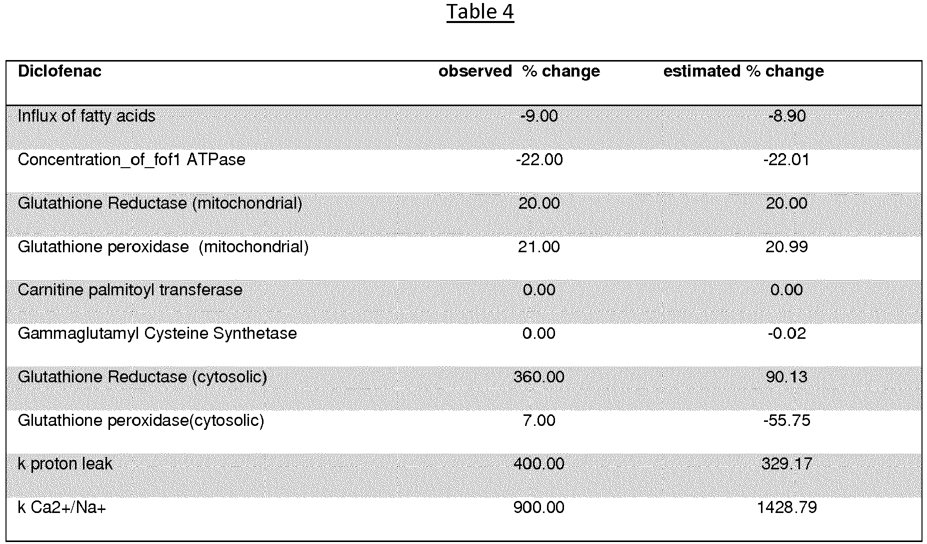

### EXAMPLE-5

### Studies with Valproic acid

Valproic acid is used in the treatment of epilepsy. It is also known to cause an inhibiton of mitochondrial beta oxidation and hence the development of fatty liver. It has been hypothesized that the acidic nature of this molecule is responsible for the onset of this phenomenon. Our method also shows that the reduction of carnitine palmitoyl transferase, a key enzyme of beta oxidation is one of the parameters altered when cells are exposed to this drug. 30050 parameter set evaluations are carried out in this case. The results are shown in table 5.

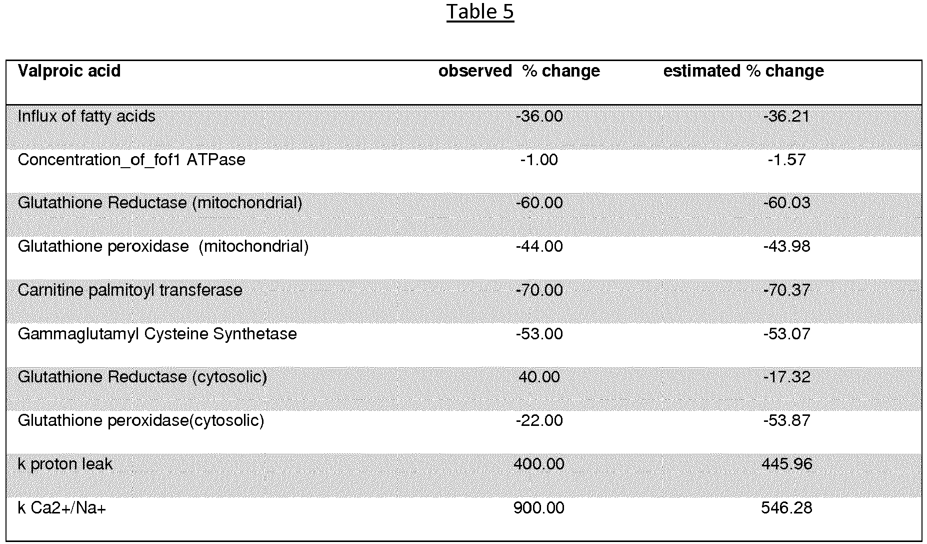

For the validation of this disclosure, biochemical enzymatic assays are used. However, methods such as NMR, or mass spectrometry can also be used for metabolite data collection. Since these can be deployed in a non-invasive and high-throughput manner, the disclosure has the potential to reduce the use of live animals in toxicological research.

## Claims

1. A method for predicting liver toxicity of a drug, said method comprising steps of:
a) obtaining metabolite profiles from untreated cells and drug-treated cells using techniques selected from a group consisting of biochemical assays, mass spectroscopy, NMR or any combinations thereof;
b) comparing the metabolite profiles obtained from the untreated cells and the drug-treated cells to determine altered metabolites 1;
c) performing Covariance Matrix Adaptive Evolutionary Strategy (CMA-ES) analysis to determine parameter set responsible for the altered metabolites 1, wherein the analysis comprises:
i. providing a parameter set to a homeostatic liver model and obtaining altered metabolites 2; and
ii. determining whether the parameter set is responsible for the altered metabolites 1 on the basis of predetermined fitness criterion; and
c) providing the parameter set as an input to a homeostatic liver model and predicting the toxicity of the drug.

2. The method as claimed in claim 1,
wherein the sub-step i of step (c) is carried out by a process comprising steps of:
a) selecting an arbitrary parameter set or a pre-defined parameter set which may be responsible for the altered metabolites 1 and providing initial values to the parameters in the set; and
b) providing the parameter set to the homeostatic liver model and simulating the homeostatic liver model to obtain altered metabolites 2;
and wherein, the sub-step ii of step (c) is carried out by a process comprising steps of:
c) determining difference between the altered metabolites 1 and the altered metabolites 2;
d) assessing whether said difference is less than or more than the predetermined fitness criterion; and
e) selecting the parameter set if the determined difference is found to be less than the predetermined fitness criterion; or discarding the parameter set if the determined difference is found to be greater than the predetermined fitness criterion and repeating the step (c) of claim 1.

3. The methods as claimed in claims 1 and 2, wherein the altered metabolites 1 and 2 are obtained by method selected from group consisting of *in*-*vitro*, and *in-silico* methods or a combination thereof.

## Patentansprüche

1. Verfahren zur Vorhersage der Lebertoxizität eines Arzneimittels, wobei das Verfahren die Schritte umfasst:
a) Erhalten von Metabolitenprofilen aus unbehandelten Zellen und arzneimittelbehandelten Zellen unter Verwendung von Techniken ausgewählt aus der Gruppe bestehend aus biochemischen Assays, Massenspektroskopie, NMR oder beliebigen Kombinationen davon;
b) Vergleichen der Metabolitenprofile, die aus den unbehandelten Zellen und den arzneimittelbehandelten Zellen gewonnen wurden, um veränderte Metaboliten 1 zu bestimmen;
c) Durchführen von Kovarianz-Matrix-adaptiver-evolutionärer Strategie (CMA-ES) Analyse, um einen Parametersatz zu bestimmen, der für die veränderten Metaboliten 1 verantwortlich ist, wobei die Analyse umfasst:
i. Bereitstellen eines Parametersatzes an ein homöostatisches Lebermodell und Erhalten von veränderten Metaboliten 2; und
ii. Bestimmen, ob der Parametersatz auf der Basis eines vorbestimmten Eignungskriteriums für die veränderten Metaboliten 1 verantwortlich ist; und
d) Bereitstellen des Parametersatzes als eine Eingabe in ein homöostatisches Lebermodell, und Vorhersagen der Toxizität des Arzneimittels.

2. Verfahren wie in Anspruch 1 beansprucht,
wobei der Unterschritt i von Schritt (c) ausgeführt wird durch ein Verfahren umfassend die Schritte:
a) Auswählen eines beliebigen Parametersatzes oder eines vorbestimmten Parametersatzes, der für die veränderten Metaboliten 1 verantwortlich sein könnte, und Bereitstellen von Anfangswerten für die Parameter in dem Satz; und
b) Bereitstellen des Parametersatzes an das homöostatische Lebermodell und Simulieren des Erhaltens veränderter Metaboliten 2 durch das homöostatische Lebermodell;
und wobei der Unterschritt ii von Schritt (c) durch ein Verfahren ausgeführt wird, das die Schritte umfasst:
c) Bestimmen der Differenz zwischen den veränderten Metaboliten 1 und den veränderten Metaboliten 2;
d) Bewerten, ob die Differenz kleiner als oder grösser als das vorbestimmte Eignungskriterium ist; und
e) Auswahl des Parametersatzes, wenn die bestimmte Differenz kleiner ist als das vorbestimmte Eignungskriterium; oder Verwerfen des Parametersatzes, wenn die bestimmte Differenz größer ist als das vorbestimmte Eignungskriterium, und Wiederholen von Schritt (c) aus Anspruch 1.

3. Verfahren wie in den Ansprüchen 1 und 2 beansprucht, wobei die veränderten Metaboliten 1 und 2 durch ein Verfahren erhalten werden, ausgewählt aus der Gruppe bestehend aus *in*-*vitro*, und *in*-*silico* Verfahren oder einer Kombination davon.

## Revendications

1. Un procédé pour prévoir la toxicité hépatique d'un médicament, ledit procédé comprenant les étapes consistant à:
a) obtenir des profils de métabolites de cellules non traitées et de cellules ayant été traitées par un médicament en faisant emploi de techniques choisies dans le groupe comprenant les essais biochimiques, la spectroscopie de masse, la RMN ou toutes combinaisons de ces techniques;
b) comparer les profils de métabolites obtenus pour les cellules non traitées et les cellules ayant été traitées par un médicament en vue de déterminer les métabolites 1 modifiés;
c) effectuer une analyse basée sur la stratégie d'adaptation de l'évolution de la matrice de covariance (CMA-ES) pour déterminer l'ensemble des paramètres responsables de la formation de métabolites 1 modifiés;
dans lequel l'analyse comprend les étapes consistant à:
i. fournir un ensemble de paramètres à un modèle de foie homéostatique et obtenir des métabolites 2 modifiés; et
ii. déterminer si l'ensemble de paramètres est responsable de la formation des métabolites 1 modifiés sur la base des critères de qualité (*fitness*) prédéterminées; et
d) fournir l'ensemble des paramètres en tant qu'apport à un modèle de foie homéostatique et prévoir la toxicité du médicament.

2. Le procédé tel que revendiqué dans la revendication 1,
dans lequel la sous-étape i. de l'étape (c) est réalisée par un procédé comprenant les étapes consistant à:
a) choisir un ensemble de paramètres arbitraires ou un ensemble de paramètres prédéfinis qui peut être responsable de la formation des métabolites 1 modifiés et donner des valeurs initiales aux paramètres de l'ensemble; et ou et fournir des valeurs initiales pour les paramètres dans l'ensemble;
b) fournir l'ensemble de paramètres au modèle de foie homéostatique et simuler le modèle de foie homéostatique pour obtenir des métabolites 2 modifiés;
dans lequel la sous-étape ii. de l'étape (c) est réalisée par un procédé comprenant les étapes consistant à:
c) déterminer la différence entre les métabolites 1 modifiés et les métabolites 2 modifiés;
d) déterminer si cette différence est inférieure ou supérieure aux critères de qualité prédéterminées; et
e) sélectionner l'ensemble des paramètres si la différence déterminée est jugée comme étant inférieure aux critères de qualité prédéterminées, ou rejeter l'ensemble des paramètres si la différence déterminée est jugée comme étant supérieure aux critères de qualité prédéterminées et répéter l'étape (c) de la revendication 1.

3. Les procédés tels que revendiqués dans les revendications 1 et 2, dans lesquels les métabolites 1 modifiés et 2 sont obtenus par un procédé choisi dans le groupe des procédés *in vitro* et *in silico* ou toutes combinaisons de ces procédés.
